# EUROPEAN PATENT SPECIFICATION

(11) **EP 4 215 139 B1**
(45) Date of publication and mention of the grant of the patent: **01.04.2026**
(21) Application number: 23152448.9
(22) Date of filing: 19.01.2023
(51) Int. Cl.: A61B 18/14, A61B 5/287, A61B 5/00, B21D 28/00

(54) **SYSTEMS AND METHODS FOR LINEAR SPINES AND SPINE RETENTION HUB FOR IMPROVED TISSUE CONTACT AND CURRENT DELIVERY**
SYSTEME UND VERFAHREN FÜR LINEARE RIPPEN UND EINE RIPPENHALTENDE NABE FÜR VERBESSERTEN GEWEBEKONTAKT UND VERBESSERTE STROMABGABE
SYSTÈMES ET PROCÉDÉS POUR COLONNES ET MOYEU DE RÉTENTION DE COLONNES POUR UN CONTACT TISSULAIRE AMÉLIORÉ ET UNE DISTRIBUTION DE COURANT AMÉLIORÉE

(30) Priority: 20.01.2022 US 202263301128 P; 14.12.2022 US 202218065994
(43) Date of publication of application: 26.07.2023
(73) Proprietor: Biosense Webster (Israel) Ltd., Yokneam 2066717 (IL)
(72) Inventor: OKARSKI, Kevin Mark, Irvine 92618 (US); DATTA, Keshava, Irvine 92618 (US); BAH, Abubakarr, Irvine 92618 (US); NGUYEN, Thanh, Irvine 92618 (US); LICHTER, Justin George, Irvine 92618 (US)
(74) Representative: Carpmaels & Ransford LLP

(56) References cited:
- US-A1- 2002 198 522
- US-A1- 2012 271 138
- US-A1- 2013 090 651
- US-A1- 2014 276 746
- US-A1- 2015 342 491
- US-A1- 2020 375 657

## Description

### FIELD

The present invention relates generally to medical devices, and in particular catheters with electrodes, and further relates to, but not exclusively, catheters suitable for use to induce irreversible electroporation (IRE) of cardiac tissues.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art tend to utilize radiofrequency (RF) electrical energy to heat tissue. RF ablation can have certain rare drawbacks due to operator's skill, such as heightened risk of thermal cell injury which can lead to tissue charring, burning, steam pop, phrenic nerve palsy, pulmonary vein stenosis, and esophageal fistula. Cryoablation is an alternative approach to RF ablation that generally reduces thermal risks associated with RF ablation but may present tissue damage due to the very low temperature nature of such devices. Maneuvering cryoablation devices and selectively applying cryoablation, however, is generally more challenging compared to RF ablation, therefore cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode catheters was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are attached in the appendix to priority application U.S. 63/301,128.

Regions of cardiac tissue can be mapped by a catheter to identify the abnormal electrical signals. The same or different catheter can be used to perform ablation. Some example catheters include a number of spines with electrodes positioned thereon. The electrodes are generally attached to the spines and secured in place by soldering, welding, or using an adhesive. Furthermore, multiple linear spines are generally assembled together by attaching both ends of the linear spines to a tubular shaft (e.g., a pusher tube) to form a spherical basket. Due to the small size of the spines and the electrodes, however, adhering the electrodes to the spines and then forming a spherical basket from the multiple linear spines can be a difficult task, increasing the manufacturing time and cost and the chances that the electrode fails due to an improper bond or misalignment. What is needed, therefore, are devices and methods of forming an improved basket assembly that can help to reduce the time required for manufacturing the basket assembly and alternative basket assembly geometries in general.
US 2020/375657 A1 describes an elongate medical device comprising an expandable structure with an expandable configuration and a collapsed configuration, a handle, operably coupled to the expandable structure, the handle further including a selective movement limiter; and a deflection control member coupled with the distal hub, where the deflection control member is configured to adjust a stiffness of the expandable structure.
US 2015/342491 A1 describes a catheter including an expandable electrode assembly having a distal cap that mechanically engages a locking feature provided on the distal ends of each of two or more flexible splines forming a portion of the expandable electrode assembly is described. The distal cap defines an atraumatic distal tip of the catheter.
US 2012/271138 A1 relates to devices, systems and methods for the detection of cardiac rhythm disorders by use of a percutaneous catheter designed to permit acquisition of numerous, simultaneous endocardial electrograms from a three dimensional array of surface electrodes, referred to as a basket style cardiac mapping catheter. The basket assembly may comprise a two-part welded distal tip with a top part and a bottom part. The distal portions of the basket splines are securably disposed within the distal tip.

### SUMMARY OF THE INVENTION

The invention is defined in independent claims 1 and 9. Advantageous embodiments of the invention are defined in the dependent claims.

### SUMMARY OF THE DISCLOSURE

Various embodiments of a medical probe and related methods are described and illustrated. There is provided, in accordance with an example of the disclosed technology, a medical probe including a tubular shaft and an expandable basket assembly. The tubular shaft can have a proximal end and a distal end. The tubular shaft extends along a longitudinal axis. The expandable basket assembly can be positioned proximate the distal end of the tubular shaft. The basket assembly can include a structure that includes spine sections and a central spine intersection, a loop retention hub, and one or more electrodes. The central spine intersection can be positioned on the longitudinal axis at a distal end of the basket assembly. Each spine section can have at least one end connected to the distal end of the tubular shaft. The loop retention hub can include a first portion and a second portion configured to mate with each other to retain a distal portion of each of the spine sections at the central spine intersection. The electrode(s) can be coupled to each of the spine sections. Each electrode can define a lumen through the electrode so that a spine section extends through the lumen of each of the one or more electrodes.

The disclosed technology can include a medical probe, comprising a tubular shaft including a proximal end and a distal end and an expandable basket assembly proximate the distal end of the tubular shaft. The basket assembly can include a plurality of spines, a loop retention hub, and one or more electrodes. The plurality of spines can each have a distal end comprising a stopper extending therefrom and a proximal end connected to the distal end of the tubular shaft. The spines can be configured to bow radially from the longitudinal axis. The loop retention hub can secure the distal ends of the plurality of spines such that the stoppers are configured to rotate with respect to the loop retention hub to allow the spines to bow radially from the longitudinal axis. The one or more electrodes can couple to each of the spines. Each electrode can define a lumen through the electrode so that a spine section extends through the lumen of each of the one or more electrodes.

The disclosed technology can include an example method of constructing a medical probe. The method can include clamping a first portion and a second portion of a loop retention hub over distal portions of a plurality of spine sections; coupling one or more electrodes to each of the spine sections; connecting at least one end of each of the spine sections to the distal end of a tubular shaft such that the plurality of spines extend along a longitudinal axis; and configuring the plurality of spine sections to extend radially outward from the longitudinal axis to define a basket shape. Each electrode can define a lumen through the electrode so that a spine section extends through the lumen of each of the one or more electrodes.

### BRIEF DESCRIPTION OF THE DRAWINGS

FIG. 1 is a schematic pictorial illustration of a medical system including a medical probe whose distal end has a basket assembly with electrodes, in accordance with an embodiment of the present invention;
FIGs. 2A and 2B are schematic pictorial illustrations showing perspective views of a medical probe in an expanded form, wherein FIG. 2A is in accordance with an embodiment of the present invention;
FIG. 2C is a schematic pictorial illustration showing a side view of a medical probe in a collapsed form, in accordance with embodiments of the present invention;
FIGs. 2D and 2E are schematic pictorial illustrations showing exploded side views of a medical probe, wherein FIG. 2D is in accordance with an embodiment of the present invention;
FIGs. 2F and 2G are schematic pictorial illustrations showing a perspective view of a medical probe in an expanded form;
FIGs. 3A and 3B are schematic pictorial illustrations showing a side view of a spine loop of a given medical device, in accordance with embodiments of the present invention;
FIGs. 4A and 4B are schematic pictorial illustration showing a side view of a loop retention hub, in accordance with an embodiment of the present invention;
FIG. 4C is a schematic pictorial illustration showing a side view of a spherical loop retention hub, in accordance with an embodiment of the present invention;
FIGs. 5A through 5C are schematic pictorial illustrations of a top-down view of various loop retention hub locking mechanisms, in accordance with an embodiment of the present invention;
FIG. 6A is a schematic pictorial illustration of a perspective view of a loop retention hub of a self-expanding basket assembly, in accordance with an embodiment of the present invention;
FIG. 6B is a schematic pictorial illustration of a perspective view of a loop retention hub of an actuated expanding basket assembly, in accordance with an embodiment of the present invention;
FIGs. 6C and 6D are schematic pictorial illustrations of side views of a self-expanding basket assembly in an expanded configuration and in a collapsed configuration, in accordance with an embodiment of the present invention;
FIGs. 6E and 6F are schematic pictorial illustrations of side views of an actuated basket assembly in an expanded configuration and in a collapsed configuration, in accordance with an embodiment of the present invention;
FIGs. 7A through 7J are schematic pictorial illustrations showing a perspective view of various example electrodes, in accordance with embodiments of the present invention;
FIGs. 8A and 8B are schematic pictorial illustrations showing various insulative jackets of a given medical device;
FIGs. 9A and 9B are schematic pictorial illustrations showing cross-sectional views of a given wire of a medical probe; and
FIG. 10 is a flowchart illustrating another method of assembling a basket assembly, in accordance with an embodiment of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

As used herein, the terms "patient," "host," "user," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example. As well, the term "proximal" indicates a location closer to the operator or physician whereas "distal" indicates a location further away to the operator or physician.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode including a high current density and high electric flux density is positioned at a treatment site, and a second electrode including comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal including a positive-voltage phase pulse (referred to herein as "positive phase") and a negative-voltage phase pulse (referred to herein as "negative phase"). "Monophasic pulse" refers to an electrical signal including only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape including an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The term "temperature rating", as used herein, is defined as the maximum continuous temperature that a component can withstand during its lifetime without causing thermal damage, such as melting or thermal degradation (e.g., charring and crumbling) of the component.

The present disclosure is related to systems, methods or uses and devices which utilize end effectors including electrodes affixed to spines. Example systems, methods, and devices of the present disclosure may be particularly suited for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation.

RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore cryoablation is not viable in certain anatomical geometries which may be reached by electrical ablation devices.

IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by programmed cell death or apoptosis, which is believed to leave less scar tissue as compared to other ablation modalities. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and methods for applying electrical signals from catheter electrodes positioned in the vicinity of myocardial tissue, preferably by applying a pulsed electric field effective to induce electroporation in the myocardial tissue. The systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U. S. Patent Publication 2021/0162210, which is attached in the appendix to priority application U.S. 63/301,128.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1, each of which is attached in the appendix to priority application U.S. 63/301,128.

To deliver pulsed field ablation (PFA) in an IRE (irreversible electroporation) procedure, electrodes should contact the tissue being ablated with a sufficiently large surface area. As described hereinbelow, the medical probe includes a tubular shaft including proximal and distal ends, and a basket assembly at the distal end of the tubular shaft. The basket assembly includes spine sections, a loop retention hub at a distal end of medical probe joining the spine sections, and at least one electrode on each spine, wherein the electrode(s) have a lumen through which a spine section extends.

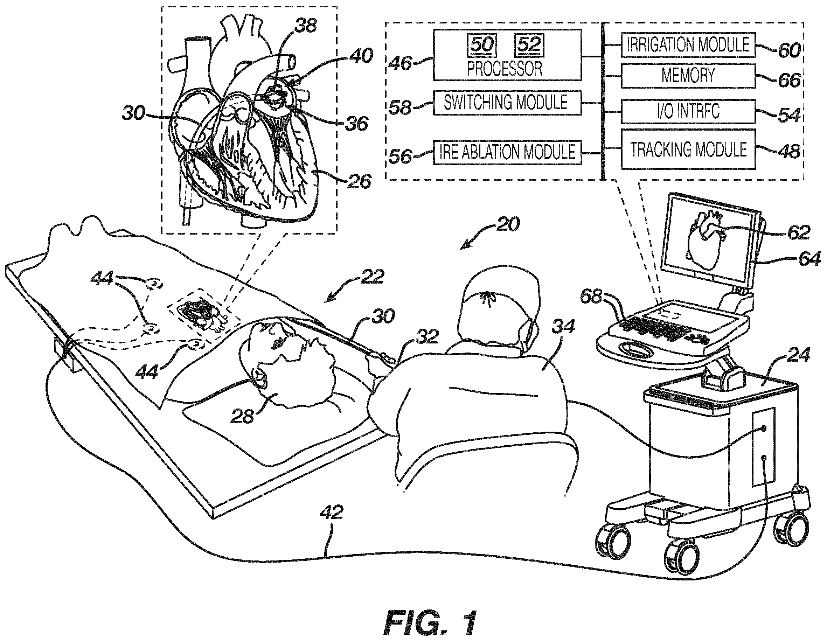

FIG. 1 is a schematic, pictorial illustration of a medical system 20 including a medical probe 22 and a control console 24, in accordance with an embodiment of the present invention. Medical system 20 may be based, for example, on the CARTO^{®} system, produced by Biosense Webster Inc. of 31 Technology Drive, Suite 200, Irvine, CA 92618 USA. In embodiments described hereinbelow, medical probe 22 can be used for diagnostic or therapeutic treatment, such as for performing ablation procedures in a heart 26 of a patient 28. Alternatively, medical probe 22 may be used, mutatis mutandis, for other therapeutic and/or diagnostic purposes in the heart or in other body organs.

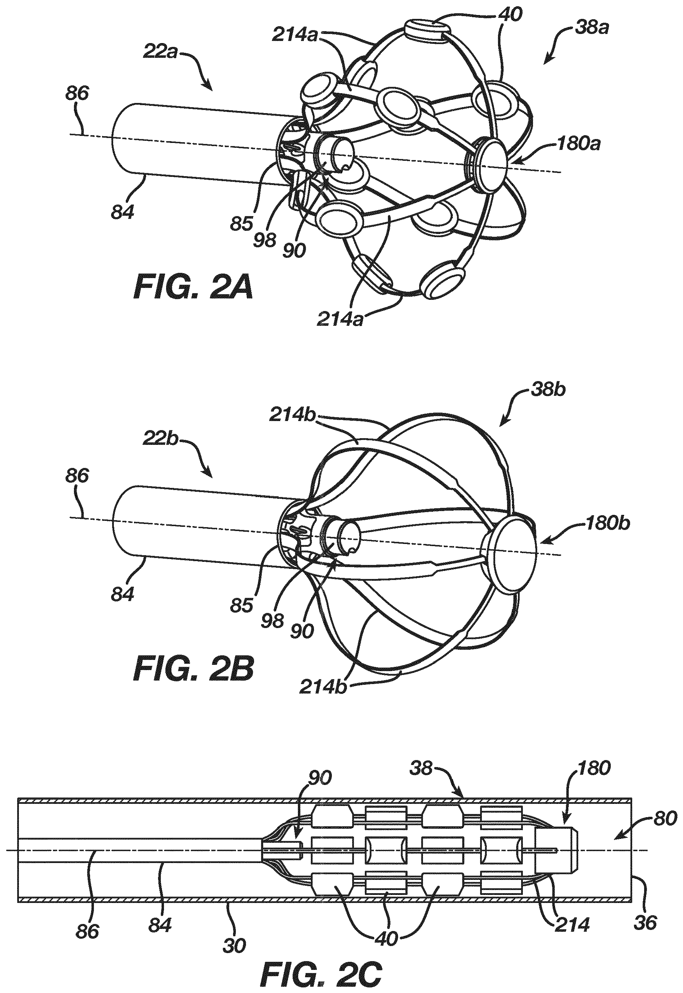

Medical probe 22 includes a flexible insertion tube 30 and a handle 32 coupled to a proximal end of the tubular shaft. During a medical procedure, a medical professional 34 can insert the medical probe 22 through the vascular system of patient 28 so that a distal end 36 of the medical probe enters a body cavity such as a chamber of heart 26. Upon distal end 36 entering the chamber of heart 26, medical professional 34 can deploy a basket assembly 38 approximate a distal end 36 of the medical probe 22. Basket assembly 38 can include a plurality of electrodes 40 affixed to a plurality of spines 214, as described in the description referencing FIGs. 2A through 2C hereinbelow. To start performing a medical procedure such as irreversible electroporation (IRE) ablation, medical professional 34 can manipulate handle 32 to position distal end 36 so that electrodes 40 engage cardiac tissue at a desired location or locations. Upon positioning the distal end 36 so that electrodes 40 engages cardiac tissue, the medical professional 34 can activate the medical probe 22 such that electrical pulses are delivered by the electrodes 40 to perform the IRE ablation.

The medical probe 22 can include a guide sheath and a therapeutic catheter, wherein the guide sheath includes the flexible insertion tube 30 and the handle 32, and the therapeutic catheter includes the basket assembly 38, electrodes 40, and a tubular shaft 84 (see FIGs. 2A through 2E). The therapeutic catheter is translated through the guide sheath so that the basket assembly 38 is positioned in the heart 26. The distal end 36 of the medical probe 22 corresponds to a distal end of the guide sheath when the basket assembly 38 is contained within the flexible insertion tube 30, and the distal end 36 of the medical probe 22 corresponds to a distal end of the basket assembly 38 when the basket assembly 38 is extended from the distal end of the guide sheath. The medical probe 22 can be alternatively configured to include a second handle on the therapeutic catheter and other features as understood by a person skilled in the pertinent art.

In the configuration shown in FIG. 1, control console 24 is connected, by a cable 42, to body surface electrodes, which typically include adhesive skin patches 44 that are affixed to patient 28. Control console 24 includes a processor 46 that, in conjunction with a tracking module 48, determines location coordinates of distal end 36 inside heart 26. Location coordinates can be determined based on electromagnetic position sensor output signals provided from the distal portion of the catheter when in the presence of a generated magnetic field. Location coordinates can additionally, or alternatively be based on impedances and/or currents measured between adhesive skin patches 44 and electrodes 40 that are affixed to basket assembly 38. In addition to being used as location sensors during a medical procedure, electrodes 40 may perform other tasks such as ablating tissue in the heart.

As described hereinabove, in conjunction with tracking module 48, processor 46 may determine location coordinates of distal end 36 inside heart 26 based on impedances and/or currents measured between adhesive skin patches 44 and electrodes 40. Such a determination is typically after a calibration process relating the impedances or currents to known locations of the distal end has been performed. While embodiments presented herein describe electrodes 40 that are preferably configured to deliver IRE ablation energy to tissue in heart 26, configuring electrodes 40 to deliver any other type of ablation energy to tissue in any body cavity is possible. Furthermore, although described in the context of being electrodes 40 that are configured to deliver IRE ablation energy to tissue in the heart 26, one skilled in the art will appreciate that the disclosed technology can be applicable to electrodes used for mapping and/or determining various characteristics of an organ or other part of the patient's 28 body.

Processor 46 may include real-time noise reduction circuitry 50 typically configured as a field programmable gate array (FPGA), followed by an analog-to-digital (A/D) signal conversion integrated circuit 52. The processor can be programmed to perform one or more algorithms and uses circuitry 50 and circuit 52 as well as features of modules to enable the medical professional 34 to perform the IRE ablation procedure.

Control console 24 also includes an input/output (I/O) communications interface 54 that enables control console 24 to transfer signals from, and/or transfer signals to electrodes 40 and adhesive skin patches 44. In the configuration shown in FIG. 1, control console 24 additionally includes an IRE ablation module 56 and a switching module 58.

IRE ablation module 56 is configured to generate IRE pulses including peak power in the range of tens of kilowatts. In some examples, the electrodes 40 are configured to deliver electrical pulses including a peak voltage of at least 700 volts (V). The medical system 20 performs IRE ablation by delivering IRE pulses to electrodes 40. Preferably, the medical system 20 delivers biphasic pulses between electrodes 40 on the spine. Additionally, or alternatively, the medical system 20 delivers monophasic pulses between at least one of the electrodes 40 and a skin patch.

Irrigation is sometimes utilized to reduce clot formation, stagnant blood flow or even reduce heat generated by ablation via the electrodes. As such, system 20 may supply irrigation fluid (e.g., a saline solution) to distal end 36 and to the electrodes 40 via a channel (not shown) in tubular shaft 84 (see FIGs. 2A through 2E). Additionally, or alternatively, irrigation fluid can be supplied through the flexible insertion tube 30. Control console 24 includes an irrigation module 60 to monitor and control irrigation parameters, such as the pressure and the temperature of the irrigation fluid.

Based on signals received from electrodes 40 and/or adhesive skin patches 44, processor 46 can generate an electroanatomical map 62 that shows the location of distal end 36 in the patient's body. During the procedure, processor 46 can present map 62 to medical professional 34 on a display 64, and store data representing the electroanatomical map in a memory 66. Memory 66 may include any suitable volatile and/or non-volatile memory, such as random-access memory or a hard disk drive.

In some embodiments, medical professional 34 can manipulate map 62 using one or more input devices 68. In alternative embodiments, display 64 may include a touchscreen that can be configured to accept inputs from medical professional 34, in addition to presenting map 62.

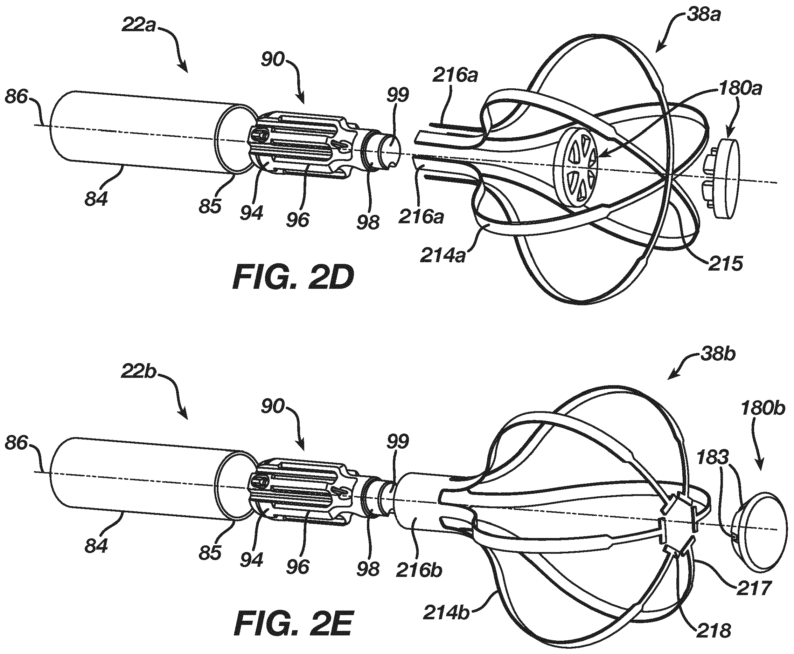

FIG. 2A is a schematic pictorial illustration showing a perspective view of a medical probe 22a including a basket assembly 38a in an expanded form when unconstrained, such as by being advanced out of an insertion tube lumen 80 (see FIG. 2C) at a distal end 36 of an insertion tube 30. The medical probe 22a illustrated in FIG. 2A lacks the guide sheath illustrated in FIG. 1. The medical probe 22a includes spine sections 214a that are retained by a loop retention hub 180a at a distal end of the basket assembly 38. The spine sections 214a include spine loops that have a distal loop 215 (see FIG. 2D) and two ends secured in the tubular shaft 84. The tubular shaft 84 is generally aligned along a longitudinal axis 86.

FIG. 2B is a schematic pictorial illustration showing a perspective view of a medical probe 22b including a basket assembly 38b in an expanded form when unconstrained, such as by being advanced out of the insertion tube lumen 80 (see FIG. 2C) at the distal end 36 of the insertion tube 30 similar to FIG. 2A except that the spine sections 214b terminate within a loop retention hub 180b that is configured to allow the spine sections 214b to rotate longitudinally with respect to the loop retention hub 180b. The spine sections 214b can be cut from a singular tube.

FIG. 2C shows a basket assembly 38 in a collapsed form that can be configured similar to the basket assembly 38a in FIG. 2A or the basket assembly 38b in FIG. 2B. The basket assembly 38 is collapsed within insertion tube 30 of the guide sheath. In the expanded form (FIGs. 2A and 2B), spines 214a, 214b bow radially outwardly and in the collapsed form (FIG. 2C) the spines 214 are arranged generally along a longitudinal axis 86 of insertion tube 30.

Referring to FIGs. 2A through 2C, during a medical procedure, medical professional 34 can deploy basket assembly 38 by extending tubular shaft 84 from insertion tube 30 causing basket assembly 38 to exit insertion tube 30 and transition to the expanded form. Spines 214 may have elliptical (e.g., circular) or rectangular (that may appear to be flat) cross-sections, and include a flexible, resilient material (e.g., a shape-memory alloy such as nickel-titanium, also known as Nitinol) forming a strut as will be described in greater detail herein.

In embodiments described herein, one or more electrodes 40 positioned on spines 214 of basket assembly 38 can be configured to deliver ablation energy (RF and/or IRE) to tissue in heart 26. Additionally, or alternatively, the electrodes can also be used to determine the location of basket assembly 38 and/or to measure a physiological property such as local surface electrical potentials at respective locations on tissue in heart 26. The electrodes 40 can be biased such that a greater portion of the one or more electrodes 40 face outwardly from basket assembly 38 such that the one or more electrodes 40 deliver a greater amount of electrical energy outwardly away from the basket assembly 38 (i.e., toward the heart 26 tissue) than inwardly. FIG. 2B is illustrated without electrodes 40 for the sake of illustration; however, the medical probe 22b includes electrodes 40 configured similarly to as illustrated in FIG. 2A.

Examples of materials ideally suited for forming electrodes 40 include gold, platinum and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 26.

FIG. 2D is an exploded view of the medical probe 22a illustrated in FIG. 2A. The electrodes 40 are omitted for the sake of illustration. The spine loops 214a include distal loops 215 that overlap within the loop retention hub 180a. The spine loops 214a include two ends 216a that are secured between the tubular shaft 84 and relief lands 96 of a spine retention hub 90 that extends longitudinally from a distal end of the tubular shaft 84 towards the distal end of basket assembly 38a.

FIG. 2E is an exploded view of the medical probe 22b illustrated in FIG. 2B. The electrodes 40 are omitted for the sake of illustration. The spine sections 214b each include a stopper 218 that is retained within the loop retention hub 180b. The spine sections 214b each include a narrow distal portion 217 that can move longitudinally within slots 183 of the loop retention hub 180b when the stopper 218 rotates to expand or contract the basket assembly 38b. The stopper 218 can have extensions that extend orthogonal to the longitudinal axis to inhibit the stopper 218 from exiting the slots 183. The spine sections 214b extend distally from a proximal tube 216b. The proximal tube 216b and the spine sections 214b can be contiguous. In some embodiments, the spine sections 214b and proximal tube 216b can be cut from a singular tube. Alternatively, the spine sections 214b can have proximal ends configured similar to the spine ends 216a illustrated in FIG. 2D.

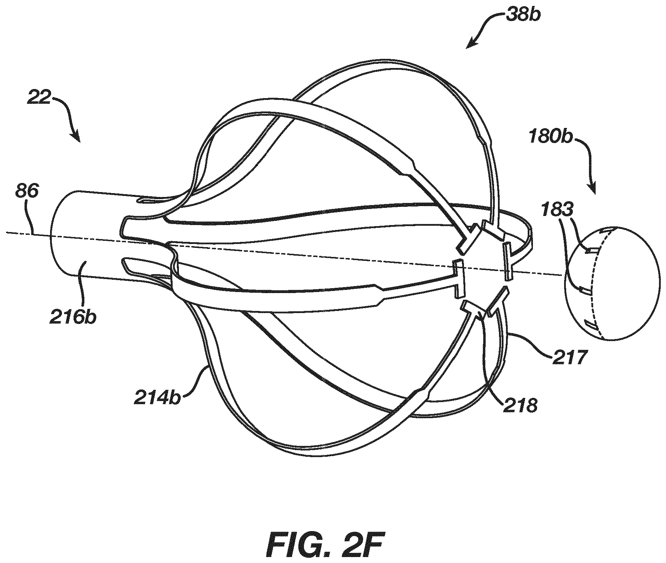

Referring collectively to FIGs. 2A through 2F, the medical probe 22 can include a spine retention hub 90 disposed proximate the distal end 85 of the tubular shaft 84. The spine retention hub 90 can be inserted into the tubular shaft 84 and attached to the tubular shaft 84. Spine retention hub 90 can include a cylindrical member 94 including a plurality of relief lands 96, multiple irrigation openings 98, and at least one spine retention hub electrode 99. Relief lands 96 can be disposed on the outer surface of cylindrical member 94 and configured to allow a portion of each spine 214, such as each spine attachment end 216a, to be fitted into a respective relief land 96. The attachment end 216 can be a generally linear end of the spine 214. The attachment end 216 can be configured to extend outwardly from the spine retention hub 90 such that the basket assembly 38 is positioned outwardly from the spine retention hub 90 and, consequently, outwardly from the tubular shaft 84. In this way, the spine 214 can be configured to position the basket assembly 38 distally from the distal end of the tubular shaft 84 and distal from the distal end of the insertion tube 30 when the basket assembly 38 is deployed. The relief lands 96 are preferably omitted when the medical probe 22b includes a proximal tube 216b joined to the spine sections 214b as illustrated in FIGs. 2E and 2F.

As described supra, control console 24 includes irrigation module 60 that delivers irrigation fluid to distal end 36 of flexible insertion tube 30. The multiple irrigation openings 98 can be angled to spray or otherwise disperse of the irrigation fluid to either a given electrode 40 or to tissue in heart 26. Since electrodes 40 do not include irrigation openings that deliver irrigation fluid, the configuration described hereinabove enables heat to be transferred from the tissue (i.e., during an ablation procedure) to the portion of the electrodes 40 on the inner side of the spines 214, and the electrodes 40 can be cooled by aiming the irrigation fluid, via irrigation openings 98, at the portion of the electrodes 40 on the inner side of the spines 214. Spine retention hub electrode 99 disposed at a distal end of retention hub 90 can be used in combination with electrodes 40 on the spines 214, or alternatively, can be used independently from electrodes 40 for reference mapping or ablation.

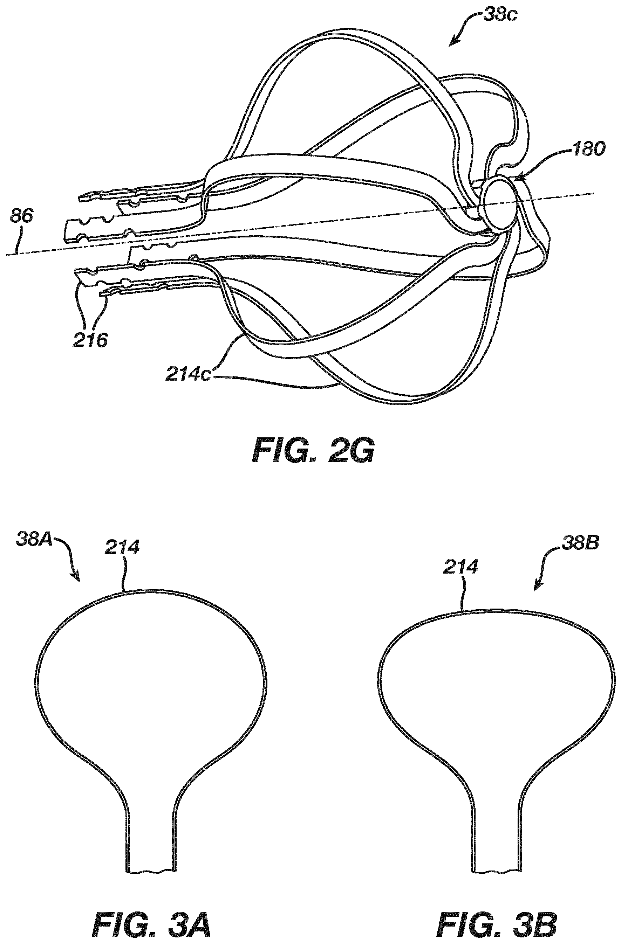

FIG. 2G illustrates a perspective view of an alternative configuration of spine sections 214c that have a curvature approximate the loop retention hub 180. As illustrated, the loop retention hub 180 can be configured similarly to the loop retention hub 180a illustrated in FIGs. 2A and 2D. Further, as illustrated, the spine sections 214c can include spine loops each including a distal loop 215 overlapped within the loop retention hub 180 and two ends that can be secured within the tubular shaft 84. Alternatively, the spine sections 214b illustrated in FIGs. 2B and 2E can be modified to include a curvature in the narrow distal portion 217 similar to the shape of the spine sections 214c illustrated in FIG. 2G.

FIGs. 3A and 3B are schematic pictorial illustrations showing a profile shape of a basket assembly 38 of a given medical device 22 when the spines sections 214 are expanded. As shown in FIG. 3A, the basket assembly 38a can be configured to form an approximately spheroid or spherical shape when in the expanded form. As another example, as shown in FIG. 3B, the basket assembly 38b can have an approximately elliptical profile and oblate-spheroid shape when in the expanded form. Although not every variation of shape is shown or described herein, one skilled in the art will appreciate that spines 214 can be further configured to form other various shapes as would be suitable for the particular application.

By including spines 214 configured to form various shapes when in the expanded form, basket assembly 38 can be configured to position the various electrodes 40 attached to spines 214 at various locations, with each location being nearer or farther from the distal end of tubular shaft 84. For example, electrode 40 attached to spine 214 illustrated in FIG. 3A near the middle of spine 214 would be farther from the distal end of tubular shaft 84 than spine 214 illustrated in FIG. 3B when basket assembly 38 is in the expanded form. In addition, each spine 214 may have an elliptical (e.g., circular) or rectangular (that may appear to be flat) cross-section, and include a flexible, resilient material (e.g., a shape-memory alloy such as nickel-titanium (also known as Nitinol), cobalt chromium, or any other suitable material).

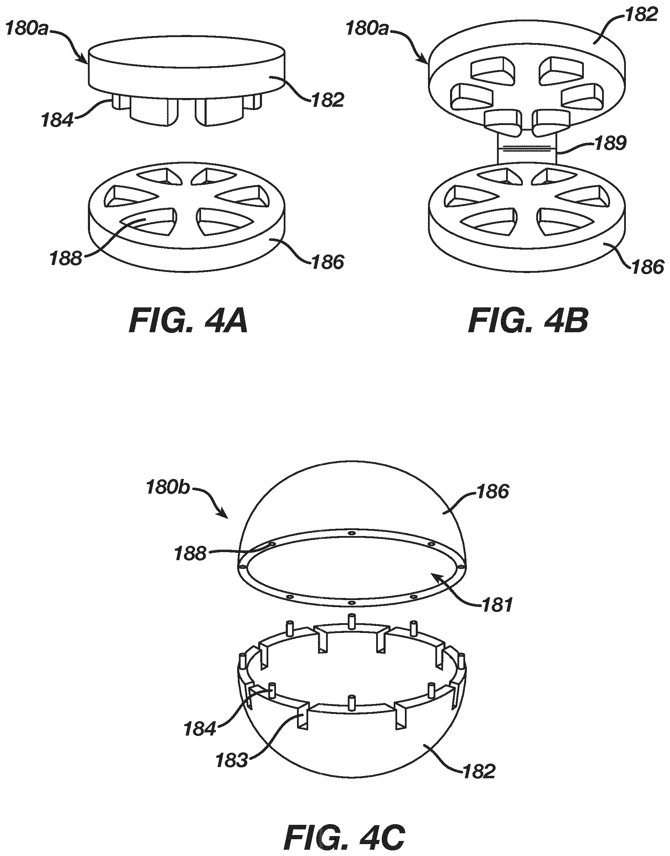

FIGs. 4A and 4B are schematic pictorial illustration showing a side view of a loop retention hub 180a configured similarly to the loop retention hub 180a illustrated in FIG. 2A and 2D. The loop retention hub 180a includes a first portion 182 including protrusions 184 and a second portion 186 including indentations 188. The protrusions 184 engage the indentations 188 to clamp the first portion 182 to the second portion 186. The loop retention hub 180a can further include a hinge 189 between the first portion 182 and the second portion 186.

FIG. 4C provides an alternative design of loop retention hub 180b configured to the loop retention hub 180b illustrated in FIG. 2F. As shown, the loop retention hub 180b is approximately spherical hollow body 181 including protrusions 184 along the first portion 182 and indentations 188 along the second portion 186. The protrusions 184 engage the indentations 188 to clamp the first portion 182 to the second portion 186. Spherical loop retention hub 180b can also include slots 183 such that at least a portion of each spine 214 may pass through. Spherical loop retention hub 180b may allow space within the hollow body 181 such that spines move more freely during deployment. Spherical loop retention hub 180b prevents the spines from bending when transitioning from the collapsed configuration to the expanded configuration. Spherical loop retention hub 180b can be used for either spines 214 with stoppers 218 configured to be positioned within the spherical loop retention hub 180b, as depicted in FIG. 2F, or alternatively can be used for spine sections 214a that have the distal loop 215 (see FIG. 2D).

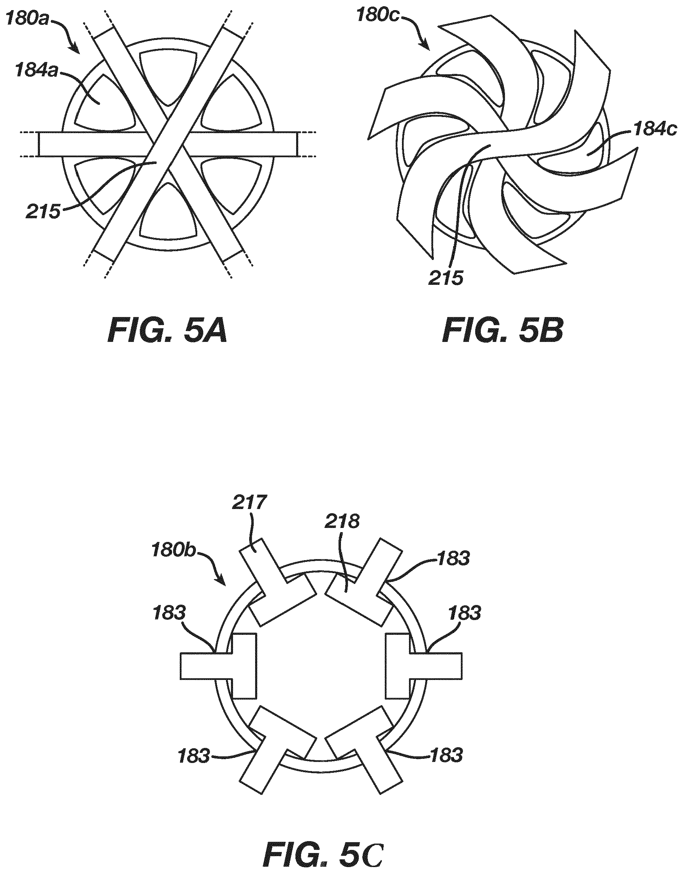

FIGs. 5A through 5C are schematic pictorial illustrations of a top-down view of various loop retention hub locking mechanisms 184a, 184b, 184c.

FIG. 5A illustrates a loop retention hub 180a configured similarly to the loop retention hub 180a illustrated in FIGs. 4A and 4B. The loop retention hub 180a includes triangular protrusions 184a (and corresponding indentations not illustrated) defining linear paths between the protrusions 184a through which the distal loops 215 extend. The distal loops 215 overlap at a central spine intersection.

FIG. 5B illustrates a loop retention hub 180c configured similarly to the loop retention hub 180a illustrated in FIGs. 4A and 4B excepting that the protrusions 184c (and corresponding indentations not illustrated) include curvature to accommodate curves of distal loops 215 through paths between the protrusions 184c. The loop retention hub 180c may be particularly suited to retain spine sections 214c including curvature as illustrated in FIG. 2F. The narrow distal portion 217 of each spine section 214b can extend through slots 183 of the loop retention hub 180c.

FIG. 5C illustrates a cross-section of the loop retention hub 180b illustrated in FIG. 2B and 2E. The stoppers 218 are positioned within the loop retention hub 180b and secured in loop retention hub 180b when clamped around the stoppers 218. The stoppers 218 are secured such that the spine sections 214b including narrow distal portion 217 can move longitudinally within slots 183 of the loop retention hub 180.

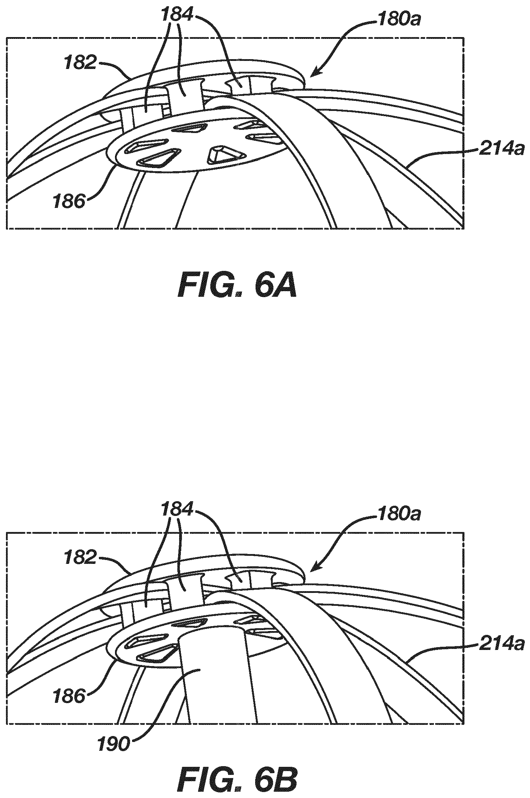

FIG. 6A is a schematic pictorial illustration of a perspective view of a loop retention hub 180a of a self-expanding basket assembly configured similarly to as illustrated in FIGs. 2A, 2D, 4A, 4B, and 5A. The basket assembly 38a is configured to self-expand upon exiting a flexible insertion tube 30 as described in relation to FIG. 2C.

FIG. 6B is a schematic pictorial illustration of a perspective view of a loop retention hub 180a of an actuated expanding basket assembly 38b. The basket assembly is configured similarly as illustrated in FIGs. 2A, 2D, 4A, 4B, and 5A excepting that the medical probe 22a further includes a central member 190 movable along the longitudinal axis 86 in relation the tubular shaft 84 to expand and collapse the basket assembly 38b. The central member 190 can include a distal end affixed to the second portion 186 of the loop retention hub 180a.

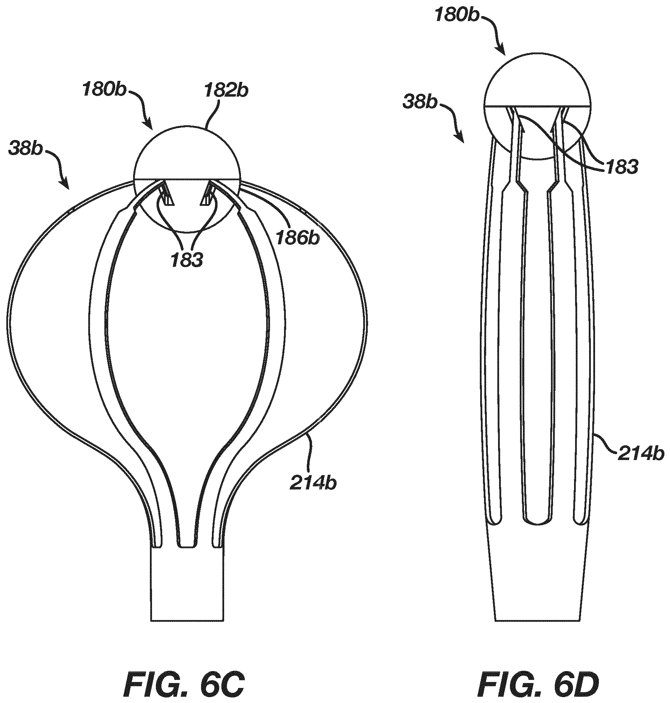

FIGs. 6C and 6D are schematic pictorial illustrations of side views of a self-expanding basket assembly 38b in an expanded configuration (FIG. 6C) and in a collapsed configuration (FIG. 6D). The self-expanding basket assembly 38b is configured similarly to as illustrated in FIGs. 2B, 2E, and 5C. The basket assembly 38b is configured to self-expand upon exiting a flexible insertion tube 30 as described in relation to FIG. 2C. The loop retention hub 180b can include a first portion 182b and a second portion 186b that when affixed together secure the stoppers 218 of the spine sections 214b.

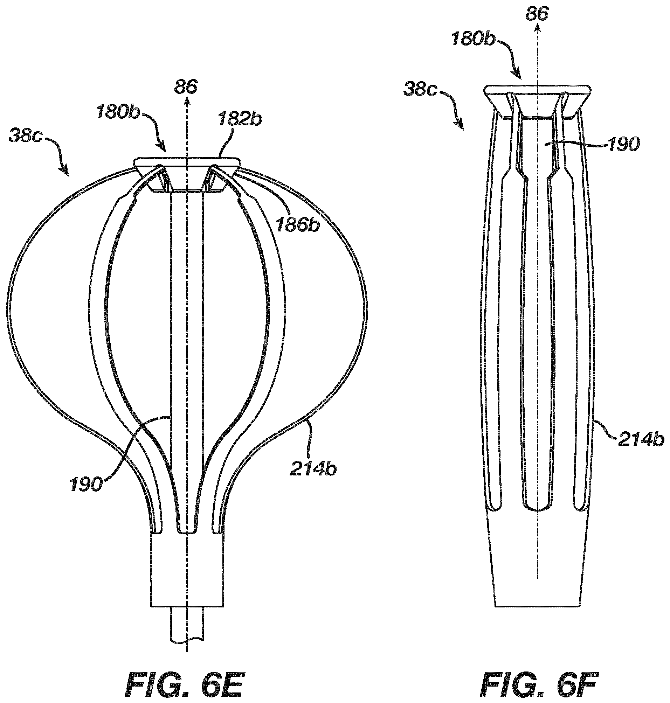

FIGs. 6E and 6F are schematic pictorial illustrations of side views of an actuated basket assembly 38c in an expanded configuration (FIG. 6E) and in a collapsed configuration (FIG. 6F). The self-expanding basket assembly 38c is configured similarly to as illustrated in FIGs. 2B, 2E, and 5C excepting that the medical probe 22b further includes a central member 190 movable along the longitudinal axis 86 in relation the tubular shaft 84 to expand and collapse the basket assembly 38c. The central member 190 can include a distal end affixed to the second portion 186b of the loop retention hub 180b.

Referring collectively to FIGs. 2A through 6F, electrodes 40 can be attached to spine sections 214 before the spine sections are inserted into the tubular shaft 84 to form the basket assembly 38. As stated previously, the spines 214 can include a flexible, resilient material (e.g., a shape-memory alloy such as nickel-titanium, also known as Nitinol) that can enable the basket assembly 38 to transition to its expanded form (as shown in FIG. 2A) when the basket assembly 38 is deployed from flexible insertion tube 30. As will become apparent throughout this disclosure, spines 214 can be electrically isolated from electrode 40 to prevent arcing from electrode 40 to the respective spine 214.

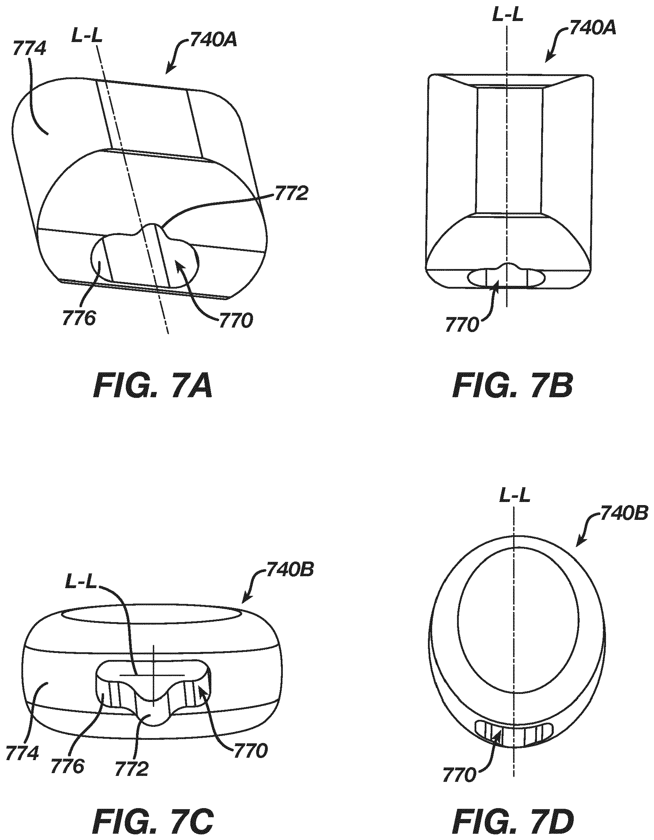

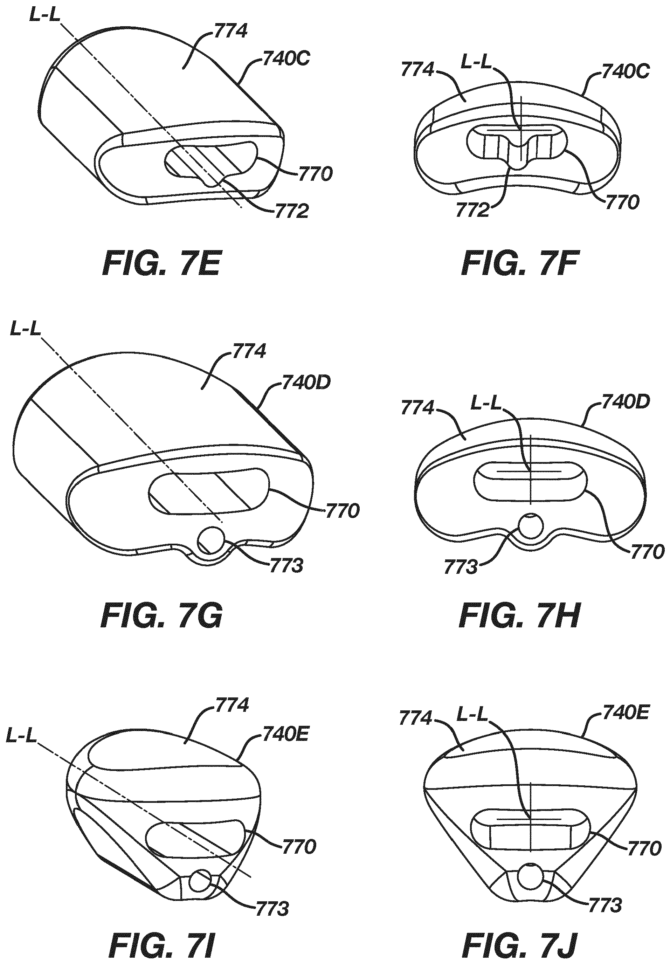

In some examples, each electrode 40 can include electrically conductive material (e.g., gold, platinum and palladium (and their respective alloys)). Turning to FIGs. 7A through 7J, electrode 40 can have a variety of cross-sectional shapes, curvatures, lengths, lumen number and lumen shape as provided as examples in electrodes 740A-740E. The electrodes 740A-740E are offered to illustrate various configurations of electrodes 40 that can be used with the medical device 22 but should not be construed as limiting. One skilled in the art will appreciate that various other configurations of electrodes 40 can be used with the disclosed technology without departing from the scope of this disclosure.

Each electrode 740A-740E can have an outer surface 774 facing outwardly from electrode 740 and an inner surface 776 facing inwardly toward electrode 740 where at least one lumen 770 is formed through electrode 740. The lumen 770 can be sized and configured to receive a spine 214 such that spine 214 can pass through electrode 740. Lumen 770 can be a symmetric opening through electrode 740A-740E and can be disposed offset with respect to a longitudinal axis L-L of the respective electrode. In other examples, lumen 770 can pass through electrode 740 in a generally transverse direction with respect to the longitudinal axis L-L of the respective electrode. Furthermore, lumen 770 can be positioned in electrode 740 nearer a bottom surface, nearer a top surface, or nearer a middle of electrode 740 depending on the particular configuration. In FIGs. 7A, 7C, and 7E through 7J, the top surface (upper side) is oriented toward the top of the drawing, the bottom surface (lower side) is oriented toward the bottom of the drawing, and the middle is between the top surface and the bottom surface. In other words, each electrode 740A-740E can include a lumen 770 that is offset with respect to a centroid of the electrode740A-740E.

In addition, as shown in FIGs. 7A through 7F, electrodes 740A-740C can have a wire relief 772 forming a recess or depression in electrode 740 adjacent lumen 770 for one or more wires to pass through lumen 770 along with a respective spine 214. Relief 772 can be sized to provide room for a wire of electrode 740 to pass through electrode 740 such that electrode 740 can be in electrical communication with the control console 24.

Alternatively, or in addition thereto, wires can pass through a wire lumen 773 as shown in example electrodes 740D and 740E in FIGs. 7G through 7J. Although not depicted, electrodes 40 may include both a wire relief 772 adjacent lumen 770 and wire lumen 773. Such electrode may permit additional wires to pass through the electrode body.

As shown in FIGs. 7A-7J, the electrodes 740A-740E can include various shapes depending on the application. For example, as illustrated in FIGs. 7A and 7B, the electrode 740A can comprise a substantially rectangular cuboid shape with rounded edges. In other examples, the electrode 740B can comprise a substantially ovoid shape (as illustrated in FIGs. 7C and 7D), the electrode 740C, 740D can have a contoured shape including a convex side and a concave side (as illustrated in FIGs. 7E through 7H), or the electrode 740E can have a contoured shape including substantially more material proximate an upper side than a lower side of the electrode 740E (as illustrated in FIGs. 7I and 7J). As will be appreciated by one of skill in the art, the various example electrodes 740A-740E shown in FIGs. 7A-7J, and described herein, are offered for illustrative purposes and should not be construed as limiting.

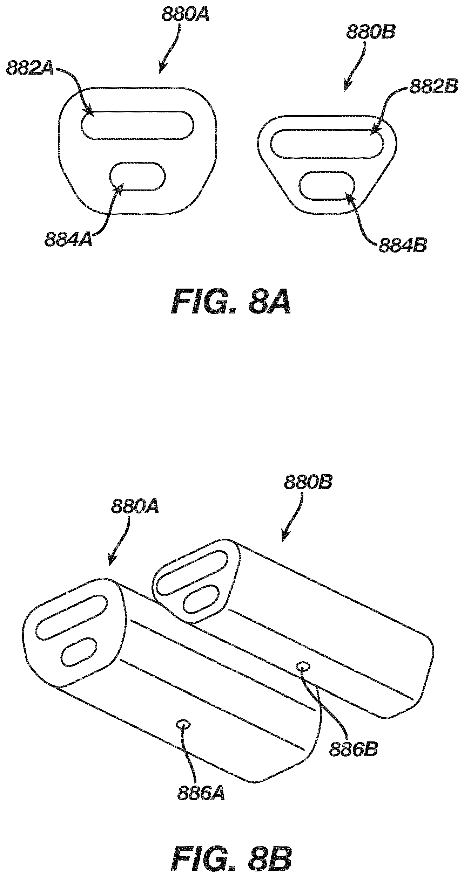

FIGs. 8A and 8B are schematic pictorial illustrations showing various insulative jackets 880A, 880B of a given medical device 22. FIG. 8A is a front view while FIG. 8B is a perspective view of insulative jackets 880A, 880B. Insulative jackets 880A, 880B can be made from a biocompatible, electrically insulative material such as polyamide-polyether (Pebax) copolymers, polyethylene terephthalate (PET), urethanes, polyimide, parylene, silicone. In some examples, insulative material can include biocompatible polymers including, without limitation, polyetheretherketone (PEEK), polyglycolic acid (PGA), poly(lactic-co-glycolic acid) copolymer (PLGA), polycaprolactive (PCL), poly(3-hydroxybutyrate-co-3-hydroxyvalerate) (PHBV), poly-L-lactide, polydioxanone, polycarbonates, and polyanhydrides with the ratio of certain polymers being selected to control the degree of inflammatory response. Insulative jackets 880A, 880B may also include one or more additives or fillers, such as, for example, polytetrafluoroethylene (PTFE), boron nitride, silicon nitride, silicon carbide, aluminum oxide, aluminum nitride, zinc oxide, and the like. Insulative jacket 880A, 880B can help to insulate a spine 214 and/or wires passing through insulative jacket 880A, 880B from electrode 40 to prevent arcing from electrode 40 to the spine 214 and/or mechanical abrasion of wires passing through insulative jacket 880A, 880B.

As illustrated in FIGs. 8A and 8B, insulative jackets 880A, 880B, can include a cross-sectional shape that is substantially trapezoidal. The insulative jacket may consist of a single lumen or multi-lumen configuration. Multi-lumen jackets may be configured such that the alloy frame and wires share a single lumen while the second lumen may be used for irrigation. The alloy frame and wires may occupy separate lumens, also, as described. The current embodiment does not utilize irrigated jackets. For these designs, the insulative jackets may be continuous (individual sleeves extending from proximal to distal end of each alloy frame strut), segmented (bridging between electrode gaps), or a combination of both. Furthermore, insulative jacket 880A, 880B can include a first lumen 882A, 882B and a second lumen 884A, 884B. First lumen 882A, 882B can be configured to receive spine 214 while second lumen 884A, 884B can be configured to receive a wire, or vice-versa. In other examples, first lumen 882A, 882B and second lumen 884A, 884B can each be configured to receive one or more wires that can be connected to one or more electrodes 40. Furthermore, as illustrated in FIG. 8B, insulative jacket 880A, 880B can include an aperture 886A, 886B through which a wire can be electrically connected to electrode 40. Although illustrated in FIG. 8B as being proximate a bottom of insulative jacket 880A, 880B, aperture 886A, 886B can be positioned proximate a top or side of insulative jacket 880A, 880B. Furthermore, insulative jacket 880A, 880B can include multiple apertures 886A, 886B with each aperture being disposed on the same side of insulative jacket (i.e., top, bottom, left, right) or on different sides of the insulative jacket depending on the application.

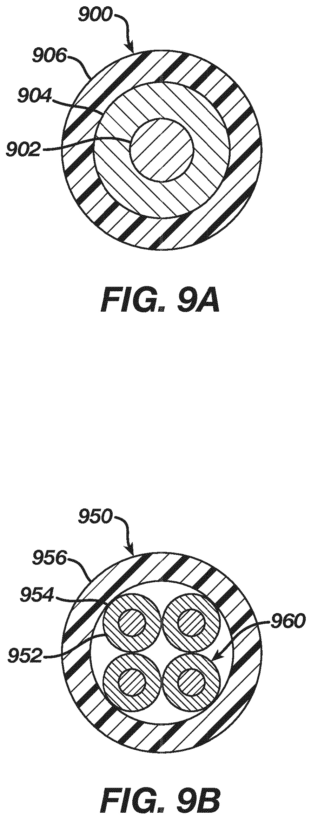

FIGs. 9A and 9B are schematic pictorial illustrations showing cross-sectional views of a given wire 900, 950 that can be connected to a given electrode 40, in accordance with an embodiment of the present invention. FIG. 9A illustrates a solid core wire 900. FIG. 9B illustrates a stranded wire 950. Each wire 900, 950 can extend through at least a portion of tubular shaft 84 and tubular shaft 84. Solid core wire 900 can include an electrically conductive core material 902 and an electrically conductive cover material 904 circumscribing electrically conductive core material 902. Likewise, stranded wire 950 can include strands each including an electrically conductive core material 952 and an electrically conductive cover material 954 circumscribing the electrically conductive core material 952. Each wire 900, 950 can include an insulative jacket 906 circumscribing the conductors. The wires 900, 950 can be configured to withstand a voltage difference of adjacent wires sufficient to deliver IRE pulses. Preferably, the wires 900, 950 can withstand at least 900V, and more preferably at least 1,800V between adjacent wires. To reduce likelihood of dielectric breakdown between conductors of adjacent wires, electrically conductive cover material 904, 954 can have a lower electrical conductivity compared to core material 902, 952.

Insulative jacket 906 can be configured to have a temperature rating between 150 and 200 degrees Centigrade so that the electrically insulative jacket 906 melts or degrades (e.g., chars and crumbles) during soldering of wire 900 to electrodes 40 (e.g., at a temperature of 300 degrees Centigrade) and therefore insulative jacket 906 of wire 900 does not need to be mechanically stripped. In other examples, insulative jacket 906 can have a temperature rating greater than 200 degrees Centigrade to prevent electrically insulating material 906 melting or degrading (e.g., charring and crumbling) during manufacture of medical probe 22 and/or during use. Insulative jacket 906 can be mechanically stripped from wire 900 prior to wires 900 being electrically connected to electrodes 40.

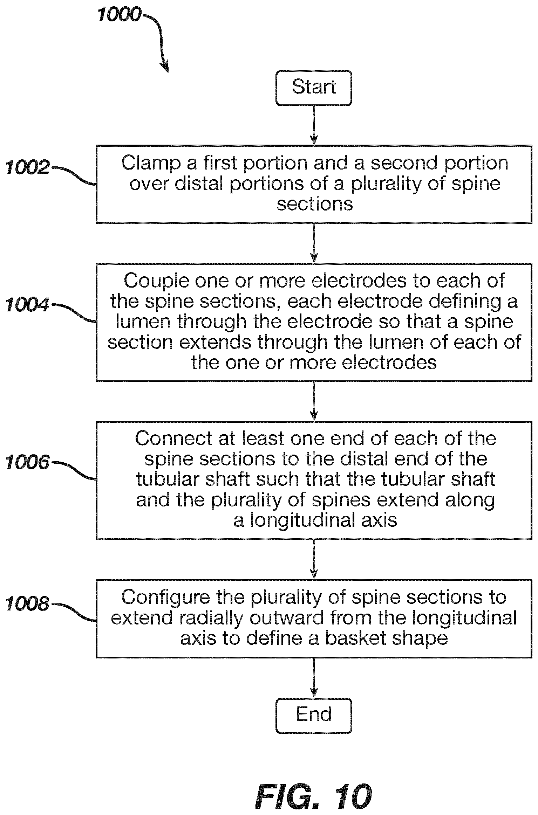

FIG. 10 is a flowchart illustrating a method 1000 of manufacturing a basket assembly 38, in accordance with an embodiment of the present invention. Method 1000 can include clamping 1002 a first portion and a second portion over distal portions of a plurality of spine sections. The method 1000 can include coupling 1004 one or more electrodes to each of the spine sections. Each electrode can define a lumen through the electrode so that the spine section extends through the lumen of each of the electrodes. The method 1000 can include connecting 1006 at least one end of each of the spine sections to the distal end of the tubular shaft such that the tubular shaft and the plurality of spines extend along a longitudinal axis. The method 1000 can include configuring 1008 the plurality of spine sections to extend radially outward from the longitudinal axis to define a basket shape.

As will be appreciated by one skilled in the art, method 1000 can include any of the various features of the disclosed technology described herein and can be varied depending on the particular configuration. Thus, method 1000 should not be construed as limited to the particular steps and order of steps explicitly described herein. It is noted that while the preference for the exemplary embodiments of the medical probe is for IRE or PFA, it is possible to also use the medical probe separately only for RF ablation (unipolar mode with an external grounding electrode or bipolar mode) or in combination with IRE and RF ablations sequentially (certain electrodes in IRE mode and other electrodes in RF mode) or simultaneously (some electrodes in IRE mode and other electrodes in RF mode).

The embodiments described above are cited by way of example, and the present invention is not limited by what has been particularly shown and described hereinabove. Rather, the scope of the invention is defined by claim 1 and includes both combinations and sub combinations of the various features described and illustrated hereinabove, as well as variations and modifications thereof which would occur to persons skilled in the art upon reading the foregoing description and which fall within the scope of claim 1.

## Claims

1. A medical probe, comprising:
a tubular shaft including a proximal end and a distal end, the tubular shaft extending along a longitudinal axis;
an expandable basket assembly (38a, 38b) proximate the distal end of the tubular shaft, the basket assembly comprising:
a structure that includes a plurality of spine sections (214a, 214b) and a central spine intersection being positioned on the longitudinal axis at a distal end of the basket assembly, each spine section including at least one end connected to the distal end of the tubular shaft,
a loop retention hub (180a, 180b) comprising a first portion and a second portion configured to mate with each other to retain a distal portion of each of the spine sections at the central spine intersection; and
one or more electrodes (40) coupled to each of the spine sections, **characterised in that**
each electrode defines a lumen through the electrode so that a spine section extends through the lumen of each of the one or more electrodes.

2. The medical probe according claim 1, further comprising a spine retention hub (90) disposed proximate the distal end of the tubular shaft, the spine retention hub comprising a cylindrical member including a plurality of relief lands (96) disposed on an outer surface of the cylindrical member to allow each spine section to be fitted into the relief land and retained therein, the spine retention hub further comprising at least one electrode disposed at a distal portion of the spine retention hub.

3. The medical probe according to claim 2, wherein the plurality of spine sections comprises spine loops (214a), each spine loop comprising a single unitary loop including a distal loop and two ends secured between the tubular shaft and in one of the relief lands of the spine retention hub.

4. The medical probe according to claim 3, wherein the distal loops of each spine loop (214a) overlap within the loop retention hub.

5. The medical probe according to claim 3 or 4, wherein the loop retention hub (180a) further comprises:
two or more protrusions positioned on the first portion or the second portion; and
two or more indentations positioned on the opposite portion of the first portion and the second portion, the indentations engaging the protrusions to clamp the first portion to the second portion.

6. The medical probe according to claim 5, wherein the plurality of spine sections fit within paths formed between the two or more protrusions.

7. The medical probe according to claim 1, wherein the loop retention hub (180b) comprises two substantially hemispherical members coupled to each other to retain the spines.

8. The medical probe according to claim 1, further comprising a central member (190) disposed within the tubular shaft and moveable along the longitudinal axis, wherein each spine loop is moveable from an expanded configuration to a collapsed configuration when the central member moves along the longitudinal axis.

9. A method of constructing a medical probe according to any preceding claim, the method comprising:
clamping a first portion and a second portion of a loop retention hub (180a, 180b) over distal portions of a plurality of spine sections;
coupling one or more electrodes (40) to each of the spine sections (214a, 214b), each electrode defining a lumen through the electrode so that a spine section extends through the lumen of each of the one or more electrodes;
connecting at least one end of each of the spine sections to the distal end of a tubular shaft such that the plurality of spines extend along a longitudinal axis; and
configuring the plurality of spine sections to extend radially outward from the longitudinal axis to define a basket shape.

10. The method of claim 9, further comprising:
affixing a spine retention hub (90) proximate a distal end of the tubular shaft, the spine retention hub comprising a cylindrical member including a plurality of relief lands (96) disposed on an outer surface of the cylindrical member, the spine retention hub further comprising at least one electrode disposed at a distal portion of the spine retention hub; and
fitting each spine section into a relief land of the plurality of relief lands.

11. The method of claim 9, wherein the plurality of spine sections (214a) comprises spine loops, each spine loop comprising a single unitary loop including a distal loop, the method further comprising one or more steps comprising:
securing two ends of each of the spine loops in the tubular shaft;
fitting the plurality of spine sections within paths formed between two or more protrusions;
overlapping the distal portions of the plurality of spine sections within the loop retention hub; and
engaging the two or more protrusions on the first portion or the second portion to two or more indentations on an opposite portion of the first portion and the second portion of the loop retention hub.

12. The method any of claims 9 to 11, further comprising:
positioning a central member within the tubular shaft such that the central member is moveable along the longitudinal axis and such that each spine loop is moveable from an expanded configuration to a collapsed configuration when the central member moves along the longitudinal axis.

## Patentansprüche

1. Medizinische Sonde, umfassend:
einen Rohrschaft, der ein proximales Ende und ein distales Ende einschließt, wobei sich der Rohrschaft entlang einer Längsachse erstreckt;
eine ausdehnbare Korbanordnung (38a, 38b), die sich nahe dem distalen Ende des Rohrschafts befindet, wobei die Korbanordnung umfasst:
eine Struktur, die eine Vielzahl von Dornabschnitten (214a, 214b) und einen Mitteldornschnittpunkt einschließt, der auf der Längsachse an einem distalen Ende der Korbanordnung positioniert ist, wobei jeder Dornabschnitt mindestens ein Ende einschließt, das mit dem distalen Ende des Rohrschafts verbunden ist,
eine Schlaufenrückhaltenabe (180a, 180b), die einen ersten Abschnitt und einen zweiten Abschnitt umfasst, die konfiguriert sind, um zueinander zu passen, um einen distalen Abschnitt jedes der Dornabschnitte an dem Mitteldornschnittpunkt zurückzuhalten; und
eine oder mehrere Elektroden (40), die mit jedem der Dornabschnitte gekoppelt sind, **dadurch gekennzeichnet, dass** jede Elektrode ein Lumen durch die Elektrode definiert, so dass sich ein Dorn durch das Lumen jeder der einen oder mehreren Elektroden erstreckt.

2. Medizinische Sonde nach Anspruch 1, ferner umfassend eine Dornrückhaltenabe (90), die nahe dem distalen Endes des Rohrschafts angeordnet ist, die Dornrückhaltenabe, die ein zylindrisches Element umfasst, das eine Vielzahl von Entlastungsflächen (96) einschließt, die auf der Außenoberfläche des zylindrischen Elements angeordnet sind, um jedem Dorn zu ermöglichen, in die Entlastungsfläche eingepasst und darin zurückgehalten zu werden, wobei die Dornrückhaltenabe ferner mindestens eine Elektrode umfasst, die an einem distalen Abschnitt der Dornrückhaltenabe angeordnet ist.

3. Medizinische Sonde nach Anspruch 2, wobei die Vielzahl von Dornabschnitten Dornschlaufen (214a) umfasst, wobei jede Dornschlaufe eine einzelne einheitliche Schlaufe umfasst, die eine distale Schlaufe und zwei Enden einschließt, die zwischen dem Rohrschaft und in einer der Entlastungsflächen der Dornrückhaltenabe gesichert sind.

4. Medizinische Sonde nach Anspruch 3, wobei sich die distalen Schlaufen jeder Dornschlaufe (214a) innerhalb der Schlaufenrückhaltenabe überlappen.

5. Medizinische Sonde nach Anspruch 3 oder 4, wobei die Schlaufenrückhaltenabe (180a) ferner umfasst:
zwei oder mehr Vorsprünge, die an dem ersten Abschnitt oder dem zweiten Abschnitt positioniert sind; und
zwei oder mehr Vertiefungen, die an dem gegenüberliegenden Abschnitt des ersten Abschnitts und des zweiten Abschnitts positioniert sind, wobei die Vertiefungen mit den Vorsprüngen in Eingriff stehen, um den ersten Abschnitt an den zweiten Abschnitt zu klemmen.

6. Medizinische Sonde nach Anspruch 5, wobei die Vielzahl von Dornabschnitten in Pfade passt, die zwischen den zwei oder mehr Vorsprüngen gebildet sind.

7. Medizinische Sonde nach Anspruch 1, wobei die Schlaufenrückhaltenabe (180b) zwei im Wesentlichen halbkugelförmige Elemente umfasst, die aneinander gekoppelt sind, um die Dorne zurückzuhalten.

8. Medizinische Sonde nach Anspruch 1, ferner umfassend ein zentrales Element (190), das innerhalb des Rohrschafts angeordnet und entlang der Längsachse beweglich ist, wobei jede Dornschlaufe von einer ausgedehnten Konfiguration zu einer zusammengeklappten Konfiguration beweglich ist, wenn sich das zentrale Element entlang der Längsachse bewegt.

9. Verfahren zum Herstellen einer medizinischen Sonde nach einem der vorstehenden Ansprüche, wobei das Verfahren umfasst:
Klemmen eines ersten Abschnitts und eines zweiten Abschnitts einer Schlaufenrückhaltenabe (180a, 180b) über distale Abschnitte einer Vielzahl von Dornabschnitten;
Koppeln einer oder mehrerer Elektroden (40) mit jedem der Dornabschnitte (214a, 214b), wobei jede Elektrode ein Lumen durch die Elektrode definiert, so dass sich ein Dornabschnitt durch das Lumen jeder der einen oder mehreren Elektroden erstreckt;
Verbinden mindestens eines Endes jedes der Dornabschnitte mit dem distalen Ende eines Rohrschafts, so dass sich die Vielzahl von Dornen entlang einer Längsachse erstreckt; und
Konfigurieren der Vielzahl von Dornabschnitten, um sich radial nach außen von der Längsachse zu erstrecken, um eine Korbform zu definieren.

10. Verfahren nach Anspruch 9, ferner umfassend:
Befestigen einer Dornrückhaltenabe (90) nahe einem distalen Ende des Rohrschafts, wobei die Dornrückhaltenabe ein zylindrisches Element einschließt, das eine Vielzahl von Entlastungsflächen (96) einschließt, die auf einer Außenoberfläche des zylindrischen Elements angeordnet sind, wobei die Dornrückhaltenabe ferner mindestens eine Elektrode umfasst, die an einem distalen Abschnitt der Dornrückhaltenabe angeordnet ist; und
Einpassen jedes Dornabschnitts in eine Entlastungsfläche der Vielzahl von Entlastungsflächen.

11. Verfahren nach Anspruch 9, wobei die Vielzahl von Dornabschnitten (214a) Dornschlaufen umfasst, wobei jede Dornschlaufe eine einzelne einheitliche Schlaufe umfasst, die eine distale Schlaufe einschließt, wobei das Verfahren ferner einen oder mehrere Schritte umfasst, die umfassen:
Sichern von zwei Enden jeder der Dornschlaufen in dem Rohrschaft;
Einpassen der Vielzahl von Dornabschnitten in Pfaden, die zwischen zwei oder mehr Vorsprüngen gebildet sind;
Überlappen der distalen Abschnitte der Vielzahl von Dornabschnitten innerhalb der Schlaufenrückhaltenabe; und
Ineingriffbringen der zwei oder mehr Vorsprünge an dem ersten Abschnitt oder dem zweiten Abschnitt mit zwei oder mehr Vertiefungen an einem gegenüberliegenden Abschnitt des ersten Abschnitts und des zweiten Abschnitts der Schlaufenrückhaltenabe.

12. Verfahren nach einem der Ansprüche 9 bis 11, ferner umfassend:
Positionieren eines zentralen Elements innerhalb des Rohrschafts derart, dass das zentrale Element entlang der Längsachse beweglich ist und derart, dass jede Dornschlaufe von einer ausgedehnten Konfiguration zu einer zusammegeklappten Konfiguration beweglich ist, wenn sich das zentrale Element entlang der Längsachse bewegt.

## Revendications

1. Sonde médicale, comprenant :
une tige tubulaire comportant une extrémité proximale et une extrémité distale, la tige tubulaire s'étendant le long d'un axe longitudinal ;
un ensemble panier (38a, 38b) expansible à proximité de l'extrémité distale de la tige tubulaire, l'ensemble panier comprenant :
une structure qui comporte une pluralité de sections colonnes (214a, 214b) et une intersection de colonnes centrale étant positionnée sur l'axe longitudinal au niveau d'une extrémité distale de l'ensemble panier, chaque section colonne comportant au moins une extrémité reliée à l'extrémité distale de la tige tubulaire,
un embout de retenue de boucle (180a, 180b) comprenant une première partie et une seconde partie conçues pour s'accoupler l'une avec l'autre afin de retenir une partie distale de chacune des sections colonnes au niveau de l'intersection de colonnes centrale ; et
une ou plusieurs électrodes (40) accouplées à chacune des sections colonnes, **caractérisée en ce que** chaque électrode définit une lumière à travers l'électrode de sorte qu'une section colonne s'étend à travers la lumière de chacune de la ou des électrodes.

2. Sonde médicale selon la revendication 1, comprenant en outre un embout de retenue de colonne (90) disposé à proximité de l'extrémité distale de la tige tubulaire, l'embout de retenue de colonne comportant un élément cylindrique comportant une pluralité de zones en relief (96) disposées sur une surface externe de l'élément cylindrique pour permettre à chaque section colonne d'être insérée dans la zone en relief et retenue à l'intérieur de celle-ci, l'embout de retenue de colonne comprenant en outre au moins une électrode disposée au niveau d'une partie distale de l'embout de retenue de colonne.

3. Sonde médicale selon la revendication 2, dans laquelle la pluralité de sections colonnes comprend des boucles de colonnes (214a), chaque boucle de colonne comprenant une boucle unitaire unique comportant une boucle distale et deux extrémités fixées entre la tige tubulaire et dans l'une des zones en relief de l'embout de retenue de colonne.

4. Sonde médicale selon la revendication 3, dans laquelle les boucles distales de chaque boucle de colonne (214a) se chevauchent à l'intérieur de l'embout de retenue de boucle.

5. Sonde médicale selon la revendication 3 ou 4, dans laquelle l'embout de retenue de boucle (180a) comprend en outre :
deux saillies ou plus positionnées sur la première partie ou la seconde partie ; et
deux renfoncements ou plus positionnés sur la partie opposée de la première partie et de la seconde partie, les renfoncements venant en prise avec les saillies pour serrer la première partie à la seconde partie.

6. Sonde médicale selon la revendication 5, dans laquelle la pluralité de sections colonnes s'insèrent à l'intérieur de chemins formés entre les deux saillies ou plus.

7. Sonde médicale selon la revendication 1, dans laquelle l'embout de retenue de boucle (180b) comprend deux éléments sensiblement hémisphériques accouplés l'un à l'autre pour retenir les colonnes.

8. Sonde médicale selon la revendication 1, comprenant en outre un élément central (190) disposé à l'intérieur de la tige tubulaire et pouvant être déplacé le long de l'axe longitudinal, dans laquelle chaque boucle de colonne peut être déplacée d'une conception expansée à une conception rétractée lorsque l'élément central est déplacé le long de l'axe longitudinal.

9. Procédé pour la construction d'une sonde médicale selon l'une quelconque des revendications précédentes, le procédé comprenant :
le serrage d'une première partie et d'une seconde partie d'un embout de retenue de boucle (180a, 180b) sur des parties distales d'une pluralité de sections colonnes ;
l'accouplement d'une ou plusieurs électrodes (40) à chacune des sections colonnes (214a, 214b), chaque électrode définissant une lumière à travers l'électrode de sorte qu'une section colonne s'étend à travers la lumière de chacune de la ou des électrodes ;
la liaison d'au moins une extrémité de chacune des sections colonnes à l'extrémité distale d'une tige tubulaire de telle sorte que la pluralité de colonnes s'étend le long d'un axe longitudinal ; et
la conception de la pluralité de sections colonnes pour s'étendre radialement vers l'extérieur à partir de l'axe longitudinal afin de définir une forme de panier.

10. Procédé selon la revendication 9, comprenant en outre :
la fixation d'un embout de retenue de colonne (90) à proximité d'une extrémité distale de la tige tubulaire, l'embout de retenue de colonne comprenant un élément cylindrique comportant une pluralité de zones en relief (96) disposées sur une surface externe de l'élément cylindrique, l'embout de retenue de colonne comprenant en outre au moins une électrode disposée au niveau d'une partie distale de l'embout de retenue de colonne ; et
l'insertion de chaque section colonne dans une zone en relief de la pluralité de zones en relief.

11. Procédé selon la revendication 9, dans lequel la pluralité de sections colonnes (214a) comprend des boucles de colonnes, chaque boucle de colonne comprenant une boucle unitaire unique comportant une boucle distale, le procédé comprenant en outre une ou plusieurs étapes comprenant :
la fixation de deux extrémités de chacune des boucles de colonnes dans la tige tubulaire ;
l'insertion de la pluralité de sections colonnes à l'intérieur de chemins formés entre deux saillies ou plus ;
le chevauchement des parties distales de la pluralité de sections colonnes à l'intérieur de l'embout de retenue de boucle ; et
la mise en prise des deux saillies ou plus sur la première partie ou la seconde partie avec deux renfoncements ou plus sur une partie opposée de la première partie et de la seconde partie de l'embout de retenue de boucle.

12. Procédé selon l'une quelconque des revendications 9 à 11, comprenant en outre :
le positionnement d'un élément central à l'intérieur de la tige tubulaire de telle sorte que l'élément central peut être déplacé le long de l'axe longitudinal et de telle sorte que chaque boucle de colonne peut être déplacée d'une conception expansée à une conception rétractée lorsque l'élément central est déplacé le long de l'axe longitudinal.
